# EUROPÄISCHE PATENTSCHRIFT

(11) **EP 1 132 104 B1**
(45) Veröffentlichungstag und Bekanntmachung des Hinweises auf die Patenterteilung: **16.11.2005**
(21) Anmeldenummer: 01105838.5
(22) Anmeldetag: 09.03.2001
(51) Int. Cl.: A61M 15/00, A61M 15/08, B05B 11/06

(54) **Spender für Medien**
Dispenser
Distributeur

(30) Priorität: 09.03.2000 DE 10011120
(43) Veröffentlichungstag der Anmeldung: 12.09.2001
(73) Patentinhaber: Ing. Erich Pfeiffer GmbH, 78315 Radolfzell (DE)
(72) Erfinder: Ritsche, Stefan, 78253 Eigeltingen (DE)
(74) Vertreter: Patentanwälte Ruff, Wilhelm, Beier, Dauster & Partner

(56) Entgegenhaltungen:
- EP-A- 0 950 423
- WO-A-91/06333
- DE-A- 4 021 263
- DE-A- 4 106 379

## Beschreibung

Die Erfindung betrifft einen Spender für in Speicherkammern eines Speicherkörpers portioniert verpackte, vorzugsweise wenigstens einen pharmazeutischen Wirkstoff enthaltende, austragbare Medien sowie eine Transporteinrichtung zur Zufuhr des Speicherkörpers. Als Medium kommen alle Arten von fließfähigen Medien, die pulver-, gasförmig und/oder flüssig sein können, in Betracht. Dabei kann es aus Dosierungs-, Lagerungs-, Hygiene- und/oder Haltbarkeitsgründen vorteilhaft sein, das Medium in Speicherkammern portioniert abzupacken, wobei in jeder Speicherkammer eine vorgegebene Menge von Medium enthalten ist. Diese Menge entspricht vorzugsweise der bei einer Applikation des Mediums auszutragenden Menge. Das Medium kann insbesondere einen pharmazeutischen Wirkstoff, beispielsweise ein Schmerz- oder Migränemittel, aber auch andere Wirkstoffe oder Wirkstoffkombinationen enthalten, die vorzugsweise durch nasale Applikation des Mediums dem Patienten verabreicht werden.

Ein gattungsgemäß zugrundegelegter Spender kann beispielsweise der DE 197 04 849 A1 entnommen werden. In dieser Druckschrift wird ein Spender zum Austragen eines Mediums, das vorzugsweise wenigstens einen pharmazeutischen Wirkstoff enthält, aus einer Speicherkammer eines Speicherkörpers beschrieben. Dabei ist das Medium in der Speicherkammer portioniert verpackt. Hierzu weist der Spender einen Stoßdorn auf, der in die Speicherkammer einführbar ist.

Ein Nachteil dabei ist es, daß die Bereitstellung der nächsten Speicherkammer durch eine Betätigung des Spenders erfolgt, die von der Betätigung zum Austragen des Mediums aus der Speicherkammer verschieden ist. Somit ist eine zweigeteilte Betätigung des Spenders erforderlich. Diese ist schlecht mit einer Einhand-Bedienbarkeit des Spenders vereinbar.

Im übrigen ist es bei dem Spender mit einer revolverartigen Bereitstellung der Speicherkammer nicht sichergestellt, daß der Bediener aus Versehen versucht, Medium aus einer bereits geleerten Speicherkammer auszutragen. Andererseits ist auch nicht sichergestellt, daß bei der Betätigung, die das Bereitstellen der nachfolgenden, noch nicht entleerten Speicherkammer dient, tatsächlich die nächste verfügbare Speicherkammer bereitgestellt, keine Speicherkammer übersprungen und ihr Inhalt daher nicht ausgetragen wird.

Der Spender nach der EP 0 950 423 A2 hat einen mit einer Luftpumpe verbundenen Drücker, der über eine Feder die Weiterschaltung einer mit Blistern bestückten Scheibe und das Öffnen eines Blisters über einen Stoßdorn auslöst. Am Ende des Betätigungsweges der Luftpumpe öffnet diese ein Ventil, das das Medium aus der geöffneten Blisteröffnung herausbläst und über den hohlen Stoßdorn austrägt. Bei diesem Spender ist es möglich, die Betätigung vorzeitig zu unterbrechen, so dass auch bei geöffnetem Blister kein Medienaustrag erfolgt.

Aufgabe der Erfindung ist es, einen Spender zu schaffen, der einhandbetätigbar und in der Lage ist, in Speicherkammern bereitgestelltes Medium zuverlässig auszutragen.

Diese Aufgabe wird bei Zugrundelegen eines gattungsgemäßen Spenders erfindungsgemäß durch die Merkmale des Anspruchs 1 gelöst.

Ein erfindungsgemäßer Spender ist dazu bestimmt, Medium auszutragen, das in Speicherkammern eines Speicherkörpers portioniert verpackt ist und vorzugsweise wenigstens einen pharmazeutischen Wirkstoff enthält. Hierzu weist der Spender einen Stoßdorn auf, der in Speicherkammern einführbar ist. Für den Spender ist ein Betätigungsmittel vorgesehen. Eine Betätigung des Betätigungsmittels im Sinne der Durchführung eines Austragvorgangs bewirkt dabei sowohl die Positionierung einer Speicherkammer in Bezug auf den Stoßdorn als auch den Austrag des Mediums aus der Speicherkammer. Bei der Betätigung des Betätigungsmittels erfolgt zunächst die Bereitstellung der nächsten, gefüllten Speicherkammer und anschließend der Austrag des Mediums aus dieser Speicherkammer.

Der Austragvorgang des Mediums aus der Speicherkammer ist in zwei Abschnitte unterteilt, wobei während des ersten Abschnittes ein Speicherelement vorgespannt wird und wobei während des zweiten Betätigungsabschnittes der eigentliche Austrag des Mediums erfolgt, wobei sich dabei das Speicherelement entspannt. Bei dem Speicherelement handelt es sich insbesondere um eine Pumpenkammer, in der während des ersten Betätigungsabschnittes ein Überdruck aufgebaut wird. Besonders vorteilhaft ist es, wenn die Pumpenkammer durch ein schaltbares Ventil verschlossen ist, wobei das Ventil beim Übergang vom ersten zu dem zweiten Betätigungsabschnitt im Öffnungssinne betätigt wird. Weiter vorteilhaft ist es, wenn der Übergang von dem ersten zum zweiten Betätigungsabschnitt des Medienaustrags wegabhängig bzgl. dem Betätigungsweg des Betätigungsmittels ist. Besonders vorteilhaft ist es weiterhin, wenn das Austragen des Mediums durch Ausblasen mittels des aus der Pumpenkammer ausströmenden Fluids erfolgt.

In vorteilhafter Ausbildung der Erfindung weist der Stoßdorn wenigstens einen ersten Strömungskanal zum Austragen des Mediums aus einer Speicherkammer des Speicherkörpers auf.

Weiter vorteilhaft ist es, wenn der Stoßdorn wenigstens einen zweiten Strömungskanal zur Zufuhr des Fluids aus der Pumpenkammer in die Speicherkammer aufweist. Dabei ist es besonders vorteilhaft, wenn der Stoßdorn zugleich als Schaltelement für das die Pumpenkammer abschließende Ventil ausgebildet ist. Gemäß einer Ausgestaltung ist der Stoßdorn so ausgebildet, daß der zweite Strömungskanal den ersten Strömungskanal ringförmig umgibt. Durch diese Gestaltung strömt das Fluid von allen Seiten gleichmäßig am äußeren Rand der Speicherkammer entlang ein, trifft sich in der Mitte am Grund der Speicherkammer und entweicht dann unter Mitnahme des auszutragenden Mediums über den ersten Strömungskanal. Dies stellt einen guten Austrag sicher.

Eine erfindungsgemäße Transportvorrichtung für einen Speicherkörper mit Speicherkammern wird durch eine Transporttrommel gebildet, die an ihrer Umfangsfläche Aufnahmen für jeweils einen Speicherkörper aufweist.

Gemäß vorteilhafter Ausgestaltung der Erfindung ist eine auf die Transporttrommel einwirkende Verdrehsperre vorgesehen. Dabei ist die Verdrehsperre so ausgebildet, daß durch ihre Wirkung eine Aufnahme für eine Speicherkammer des Speicherkörpers in seiner bzgl. dem Stoßdorn ausgerichteten Lage gehalten ist.

In vorteilhafter Weise ist eine Rückdrehsperre am Spender ausgebildet, die auf die Transporttrommel einwirkt und die so ausgestaltet ist, daß sie eine Drehbewegung der Transporttrommel in einer Transportrichtung erlaubt und eine Drehbewegung entgegen der Transportrichtung sperrt.

Gemäß vorteilhafter Ausgestaltung weist die Transporttrommel an ihrer Stirnseite Mitnehmer auf. Vorzugsweise ist jeder Aufnahme der Umfangsfläche der Transporttrommel stirnseitig ein Mitnehmer zugeordnet. Als Betätigungsmittel kann eine Hakenklinge dienen, die einen der Mitnehmer hintergreift und dadurch den Transport der Transporttrommel um eine Speicherkammer übernimmt. Bei der Rückbewegung der Hakenklinge kann die Hakenklinge axial bezüglich der Transporttrommel versetzt an Mitnehmern vorbeigeführt werden. Dies kann sowohl durch eine entsprechende Führungskulisse für die Hakenklinge selbst als auch dadurch erfolgen, daß die Mitnehmer elastisch an der Transporttrommel befestigt sind und während der Rückbewegung durch die Hakenklinge aus dem Bewegungsraum verdrängt werden können.

Weiter vorteilhaft ist es, wenn das Betätigungsmittel wenigstens einen auf Mitnehmer einwirkenden Schieber aufweist. Gemäß vorteilhafter Ausgestaltung kann der Schieber zugleich als Verdrehsperre ausgebildet sein. Er kann ferner in vorteilhafter Weise gleichzeitig auch als Rückdrehsperre ausgebildet sein.

Weiter vorteilhaft ist es, wenn der Schieber mittels dem Betätigungsmittel verschiebbar und so geführt angeordnet ist, daß er entlang der Mitnehmer aufweisenden Stirnseite auf einer Kreissehne an der Transporttrommel vorbei bewegt wird. Dabei kann es weiter vorteilhaft sein, wenn die Mitnehmer als in axialer Richtung von der Stirnfläche abragende, vorzugsweise zylindrisch ausgebildete Erhebungen ausgebildet sind, die konzentrisch zur Drehachse der Transporttrommel auf der Stirnfläche angeordnet sind. Weiter vorteilhaft ist es, wenn die Mitnehmer auf in der Stirnfläche ausgebildeten Materialzungen angeordnet sind. Die Materialzungen sind dabei vorzugsweise U-förmig ausgebildet und in Drehrichtung der Transporttrommel mit der Stirnfläche verbunden. Dabei sind die Materialzungen derart ausgebildet, daß sie um die Höhe der jeweiligen Erhebung entgegen der dabei entstehenden Biegekräfte in Richtung auf das Innere der Transporttrommel reversibel verbiegbar sind.

Gemäß bevorzugter Ausgestaltung der Erfindung ist der Schieber im wesentlichen balkenförmig ausgebildet. Seine Längskante erstreckt sich dabei in Richtung der Kreissehne entlang derer der Schieber bzgl. der Transporttrommel beweglich ist. Die Stirnseite des Schiebers ist dabei auf einen Mitnehmer hin ausgerichtet. Während eines Betätigungsvorganges gelangt der Schieber wenigstens zeitweise mit seiner Stirnseite in Anlage mit einem Mitnehmer.

Eine weitere vorteilhafte Ausgestaltung des Schiebers sieht vor, daß dieser eine Ausnehmung aufweist. Dabei ist die Ausnehmung auf der der Stirnseite der Transporttrommel zugewandten Seite ausgebildet. Sie erstreckt sich auch in den Bereich der den Mitnehmern zugewandten Längskante des Schiebers. Die Ausnehmung dient dabei der Aufnahme des Mitnehmers, der auf den Mitnehmer folgt, welcher bei einer Betätigung mit der Stirnseite des Schiebers in Anlage gelangt.

Vorzugsweise ist die in Bewegungsrichtung des Schiebers gesehen hintere, parallel zur Stirnseite des Schiebers ausgerichtete, die Ausnehmung begrenzende Anschlagfläche in ihrer Lage bzgl. der Stirnkante und den Mitnehmern derart ausgebildet, daß eine in Transportrichtung der Transporttrommel gerichtete kraftschlüssige Verbindung zwischen einem in der Aufnahme befindlichen Mitnehmer und der Anschlagfläche herstellbar ist.

Gemäß weiter vorteilhafter Ausgestaltung ist die Ausnehmung an der in Bewegungsrichtung vorderen, der Anschlagfläche gegenüberliegenden und die Ausnehmung begrenzenden Seite rampenförmig ausgebildet.

Gemäß weiter vorteilhafter Ausgestaltung kann die Rückdrehsperre dadurch zumindest teilweise gebildet werden, daß auf der dem Schieber diametral gegenüberliegenden Seite der Transporttrommel eine Rückdrehsperre vorgesehen ist, deren Sperrwirkung durch Kraftschluß zwischen einem transporttrommelseitigen Mitnehmer und einer gehäusefesten Anlagefläche erreicht wird, wobei die Anlagefläche in Transportrichtung der Transporttrommel überfahrbar ist.

Gemäß vorteilhafter Ausgestaltung der Erfindung ist der Speicherkörper als Blisterstreifen ausgebildet, der eine Mehrzahl von Speicherkammern mit austragbarem Medium aufweist. Dabei ist es weiter vorteilhaft, wenn der Blisterstreifen in Form eines Trommelspeichers bereitgestellt wird. Dabei weist der Speicherkörper eine gleichmäßig beabstandete Anordnung von Speicherkammern auf. Dabei ist jede Speicherkammer für sich genommen vorzugsweise hermetisch verschlossen. Dabei ist das die Speicherkammer verschließende Material zumindest abschnittsweise so ausgebildet, daß es von dem Stoßdorn durchstechbar ist.

Im übrigen ist die Erfindung nachfolgend anhand der in der Zeichnung dargestellten Ausführungsbeispiele näher erläutert. Dabei zeigt:
- Fign. 1 bis 3: eine schematische Schnittdarstellung einer ersten Ausführungsform der Erfindung;
- Fig. 4: die schematische Darstellung einer zweiten Ausführungsform in teilgeschnittener Darstellung;
- Fig. 5: eine schematische Querschnittdarstellung eines erfindungsgemäßen Spenders im Bereich des Austragkanals und der Speicherkammer, aus der gerade Medium ausgetragen wird;
- Fig. 6: eine vergrößerte schematische Querschnittsdarstellung des Schiebers der Transporttrommel;
- Fign. 7a bis 7c: eine alternative Ausgestaltung von Nasenadapter und Stoßdorn; und
- Fig. 8: eine teilgeschnittene Darstellung einer weiteren Ausführungsform

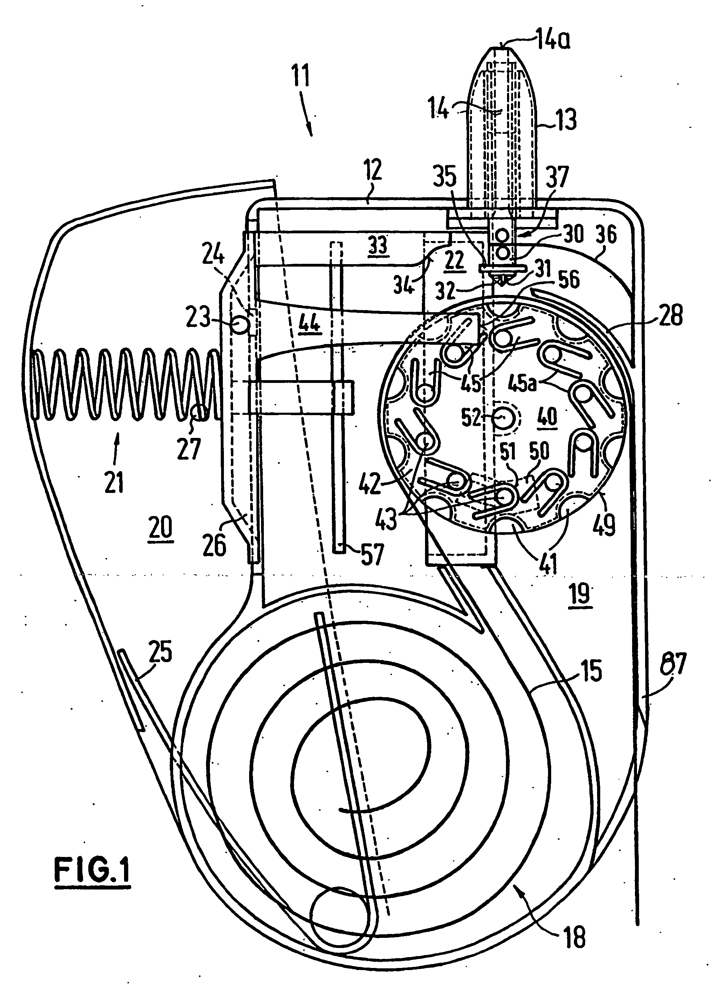

Die Fign. 1 bis 3 zeigen in schematischer Schnittdarstellung eine erste Ausführungsform der Erfindung.

Der Spender 11 wird in seiner Außenkontur durch das Gehäuse 12, den Nasenadapter 13 sowie das Betätigungsmittel 20, einem Betätigungshebel, bestimmt. Der Nasenadapter 13 dient der nasalen Wirkstoffapplikation beim Patienten. An seinem dem Gehäuse 12 abgewandten, vorderen Ende weist der Nasenadapter 13 eine Düse 14a für den Austritt des auszutragenden Medium auf. Spenderseitig mündet der Austragkanal 14 des Nasenadapters 13 an der Düse. Axial beweglich und entsprechend geführt ist der Stoßdorn 30 so im Gehäuse angeordnet, daß er mit seinem düsenseitigen Ende in den Nasenadapter 13 hineinragt. Der Stoßdorn 30 ist an seinem speicherkammerseitigen Ende so ausgebildet, daß er dazu geeignet ist, die Abdeckung der Speicherkammer 16 zu durchstoßen. Dazu weist der Stoßdorn 30 zum Beispiel zwei rechtwinklig über Kreuz liegende, kegelförmig auf die Speicherkammer 16 hin ausgerichtete Schneidkanten auf. Diese sind fest und schneidend genug, um das vorzugsweise als Deckel der Speicherkammer verwendete Folienmaterial, das aus beispielsweise metallbedampfter Kunststofffolie bestehen kann, so durchtrennt, daß bei dem Trennvorgang keine Spanbildung des Folienmaterials auftritt. Das Fehlen der Spanbildung ist insofern wichtig, als Späne dazu geeignet sein könnten, den Austragkanal 14 zumindest teilweise zu blockieren und somit den Austrag der vorgegebenen Menge an Medium unbeabsichtigt reduzieren könnte, so daß die vorbestimmte, portionierte Menge Wirkstoff nicht mehr appliziert werden kann. Zudem soll das die Speicherkammer zuverlässig an der Einstichstelle des Stoßdorns geöffnet werden. Besonders vorteilhaft ist es, wenn das durchstochene Material dichtend an dem in die Speicherkammer eingedrungenen Stoßdorn anliegt.

Daneben sind an dem Stoßdorn 30 noch der Anlagering 35 ausgebildet, der einerseits der flächenbündigen Anlage an die Oberfläche des durchstochenen Deckels der Speicherkammer 16 dienen kann und der andererseits auch im Zusammenwirken mit dem Schaltschieber 33 zum Betätigen des Stoßdorns, nämlich zum Hinunterdrücken des Stoßdorns 30 in Richtung auf die Transporttrommel 40 mit der Aufnahme 41, in der sich eine Speicherkammer 16 des Blisterstreifens 15 befindet, dienen kann. Damit der Stoßdorn in seiner unbetätigten Ruhelage nicht in Eingriff mit dem Blisterstreifen 15 gelangt, ist eine einerseits am Gehäuse 12 befestigte und andererseits in einer entsprechend ausgebildeten Aufnahme 37 befestigten Blattfeder 36 vorgesehen, die den Stoßdorn 30 in Richtung auf die Düse 14a hin beaufschlagt. Die Federkraft ist dabei so gewählt, daß der Stoßdorn 30 unbetätigt sicher in seiner in Fig. 1 dargestellten Ruhelage gehalten wird und daß andererseits die über den Schaltschieber 33 aufzubringenden Betätigungskräfte nicht über ein bedienbares Maß hinausgehen.

Die in den Fign. 1 bis 3 nicht dargestellten Speicherkammern des Blisterstreifens 15 werden von den Aufnahmen 41 der Transporttrommel 40 aufgenommen und darin wenigstens in Drehrichtung der Trommel ausgerichtet gehalten. Der Blisterstreifen 15 wird dabei vorzugsweise in Form eines Trommelspeichers 18 bevorratet, so daß eine größere Blisterstreifenlänge und damit eine größere Anzahl an Speicherkammern bevorratet werden kann. Der Durchmesser des Trommelspeichers 18, insbesondere der Innendurchmesser des aufgewickelten Blisterstreifens ist dabei durch die Materialsteifigkeit des Blisterstreifens selber begrenzt. Der Blisterstreifen wird von dem bevorrateten Material im Laufe der Abfolge von Betätigungen des Spenders schrittweise abgewickelt und zunächst zu der Transporttrommel 40 geführt, die zumindest teilweise von dem Blisterstreifen 15 umschlungen wird. Dabei müssen der Abstand zwischen den Speicherkammern auf dem Blisterstreifen und der Abstand zwischen den Aufnahmen 41 an der Umfangsseite 49 der Transporttrommel 40 aufeinander abgestimmt sein. Der Umschlingungsbereich der Transporttrommel 40 durch den Blisterstreifen 15 erstreckt sich dabei zumindest über den Abschnitt, der dem Stoßdorn 30 gegenüberliegt. Im Umschlingungsbereich ist es dabei vorteilhaft, wenn zumindest abschnittsweise Leitmittel 28 vorgesehen sind, die das radiale Spiel des Blisterstreifens 15 gegenüber der Transporttrommel 40 begrenzen. Nach dem Umschlingungsbereich verläßt der Blisterstreifen 15, der nunmehr nur noch entleerte Speicherkammern aufweist, das Gehäuse 12 durch den Ausfuhrschacht 19. Am Ende des Ausfuhrschachtes 19 kann eine Abreißkante 87 vorge sehen sein, damit Abschnitte des überstehenden Blisterstreifens abgerissen und entsorgt werden können.

Zur Betätigung des Spenders 11 ist das Betätigungsmittel 20 vorgesehen, das durch die Einwirkung der Rückstellfeder 25 in seiner unbetätigten Ruhestellung gehalten wird. Durch das Betätigen des Betätigungsmittels 20 ist das Speicherelement 21, im vorliegenden Fall ein Federspeicher, vorspannbar. Dabei stützt sich der Federspeicher 21 einerseits am Betätigungsmittel 20 und andererseits an einer Trägerplatte 26 ab. Von der Trägerplatte 26 ragt dabei sowohl der Schaltschieber 33 als auch der Schieber 44, der auf die Mitnehmer 43 der Transporttrommel 40 einwirkt, ab. Des weiteren ist an der Trägerplatte 26 ein Anschlagstift 23 ausgebildet, der mit einem am Betätigungsmittel 20 vorgesehenen Anschlagelement 24 zusammenwirkt, wobei die beiden Elemente zur Begrenzung des Relativweges zueinander dienen.

Die Transporttrommel 40 weist an ihrer Umfangsseite 49 Aufnahmen 41 für Speicherkörper auf. An wenigstens einer ihrer Stirnseiten 42 weist sie konzentrisch zu ihrer gehäusefest angeordneten Drehachse 52 angeordnete Mitnehmer 43 auf. Die Mitnehmer 43 sind dabei als gegenüber der Stirnseite 42 zylinderförmig ausgebildete Erhebungen. Dabei ist jeder Aufnahme 41 für eine Speicherkammer ein Mitnehmer 43 zugeordnet. Die Mitnehmer 43 sind dabei auf Materialzungen 45 angeordnet, die in der Stirnseite 42 ausgebildet sind. Dabei trennt ein U-förmiger Schlitz 45a die Materialzunge 45 von der restlichen Fläche der Stirnseite 42. Die Mitnehmer 43 sind jeweils am freien Ende der Materialzungen 45 angeordnet, an dem diese nicht mit der Stirnseite 42 verbunden sind. Damit die Transporttrommel 40 gegen ein Zurückdrehen gesichert ist, ist gehäuseseitig ein Rückdrehsperrelement 50 ausgebildet, das mit den Mitnehmern 43 zusammenwirkt. Bei einer Drehung der Transporttrommel im Sinne des Bereitstellens der nächstfolgenden Speicherkammer an der bzgl. dem Stoßdorn 30 ausgerichteten Position kann das vorzugsweise rampenförmig ausgebildete Rückdrehsperrelement dadurch überfahren werden, daß der Mitnehmer entgegen der Wirkung der Materialelastizität der Materialzunge 45 in die Ebene der Stirnseite 42 in axialer Richtung der Drehachse 52 hineingedrückt wird. An dem Rückdrehsperrelement ist eine Anlagefläche 51 ausgebildet, die im wesentlichen die Außenkontur eines Mitnehmers 43 aufweist und die die Rampe des Rückdrehsperrelementes begrenzt. Es ist somit eine Kante an der Rampe ausgebildet, hinter der Mitnehmer 43 aufgrund der Materialelastizität der Materialzunge in seine ursprüngliche Lage zurückspringt und dann aufgrund der Anlage an der Anschlagfläche 51 nicht mehr in der Lage ist, in die Gegenrichtung gedreht zu werden. Dabei muß die Lage des Rückdrehsperrelementes 50 bzgl. der Lage des Stoßdorns 30 so angeordnet sein, daß dann, wenn ein Mitnehmer 43 das Rückdrehsperrelement mit seiner Anlagefläche 51 gerade überfahren hat, eine Aufnahme 41 für einen Speicherkörper 16 sich genau in der Lage befindet, in der es bzgl. dem Stoßdorn 30 ausgerichtet ist. Vorzugsweise ist das Rückdrehsperrelement genau diametral dem Stoßdorn 30 gegenüberliegend angeordnet.

Die Fig. 1 zeigt den Spender 11 in seiner unbetätigten Ausgangsstellung. Wird der Spender nun an seinem Betätigungsmittel 20 betätigt, so wird zunächst das Speicherelement 21, ein Federspeicher, vorgespannt. Der Federspeicher 21 ist dabei zwischen der Trägerplatte 26 und dem Betätigungsmittel 20 angeordnet. Dabei wird die Trägerplatte 26 zu diesem Zeitpunkt noch von einer Verrastung mit dem Gehäuse 12 lagefest bzgl. dem Gehäuse 12 gehalten. Während dieser Betätigung wird also lediglich der Federspeicher 21 und die Rückstellfeder 25 vorgespannt.

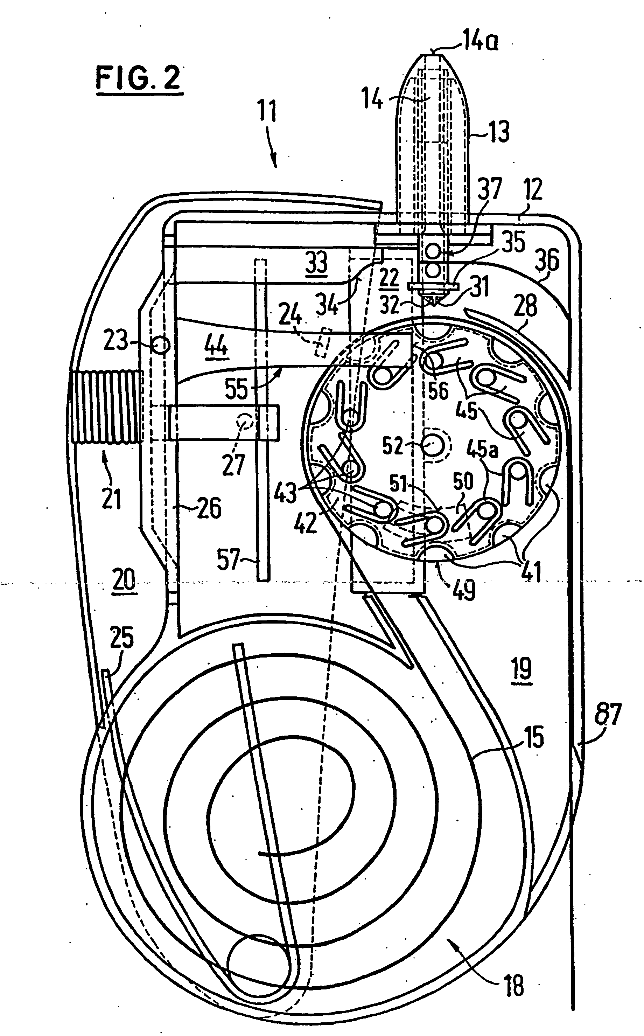

Auf diese Weise wird die in der Fig. 2 dargestellte Stellung erreicht. In der in der Fig. 2 dargestellten Stellung ist sowohl die Rückstellfeder 25 als auch der Federspeicher 21 vorgespannt. Die seitens des Betätigungsmittels 20 ausgebildete Schaltnocke 27 hat fast die Lage erreicht, in der sie die Verrastung zwischen Trägerplatte 26 und Gehäuse 12 löst. Dabei hat das Betätigungsmittel 20 schon nahezu seine Betätigungsendlage erreicht. Im weiteren Bewegungsablauf wird sie noch bis zur Betätigungsendlage weiterbewegt, in der dann die Schaltnocke 27 die Rastverbindung zwischen der Trägerplatte 26 und dem Gehäuse 12 löst. Diese Vorgehensweise hat den Vorteil, daß der nach dem Lösen dieser Rastverbindung stattfindende Austragvorgang allein aufgrund der Wirkungen des Kraftspeichers 21 erfolgt und somit ein Einwirken des Benutzers zu diesem Zeitpunkt nicht mehr erforderlich ist. Dabei kann es vorrteilhaft sein, wenn gleichzeitig mit dem Lösen der Rastverbindung zwischen Trägerplatte 26 und Gehäuse 12 eine Rastverbindung zwischen Gehäuse 12 und dem Betätigungsmittel 20 hergestellt wird, so daß das Betätigungsmittel in unveränderter Lage gehalten wird und somit zu diesem Zeitpunkt noch kein Rückstellvorgang des Betätigungsmittels 20 durchgeführt wird. Auf diese Weise ist der Betätigungsablauf für einen Austrag und den Transport des Blisterstreifens mittels der Transporttrommel 40 komplett unabhängig vom Ablauf der Betätigung des Betätigungsmittels 20. Insbesondere ist es dem Benutzer nicht möglich, nur Teilbetätigungen oder andere unsachgemäße Betätigungen durchzuführen. Er ist gezwungen, das Betätigungsmittel 20 bis zu seiner Endlage zu betätigen, wobei es auf die Art der Betätigung nicht ankommen, da die im Federspeicher gespeicherte Energie unabhängig vom Ablauf der Betätigung des Betätigungsmittels 20 ist. Nur aufgrund der hier zwischengespeicherten Energie wird der gesamte Austragvorgang durchgeführt.

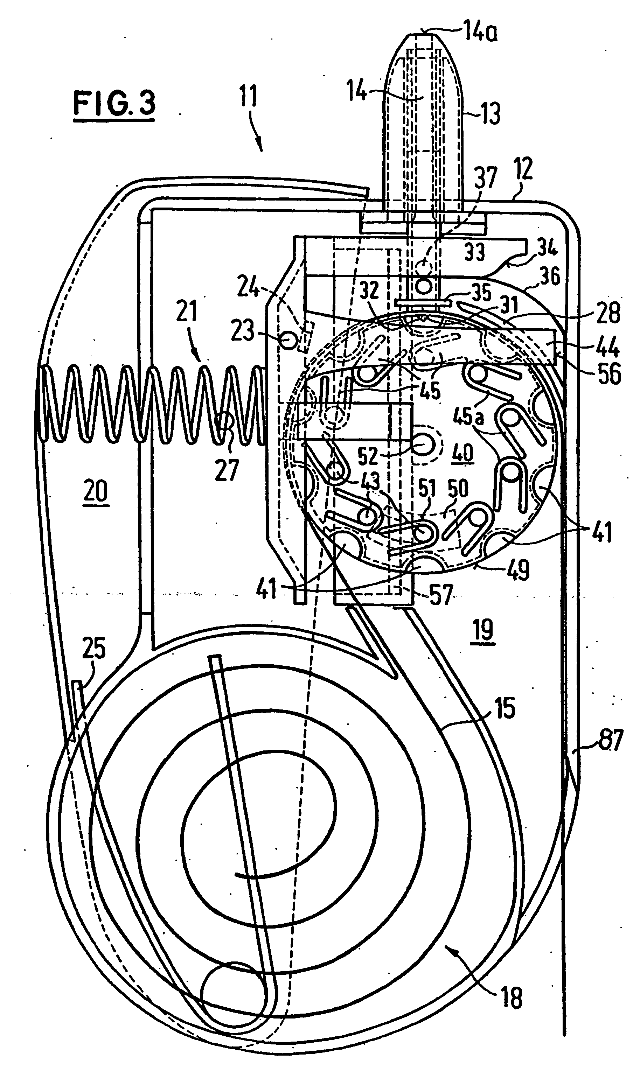

Von der in der Fig. 2 dargestellten Stellung gelangt man während des Ablaufes eines Betätigungsablaufes in die in der Fig. 3 dargestellte Stellung.

Die Fig. 3 zeigt die Lage der Teile zueinander am Ende eines Austragvorganges, bevor das Betätigungsmittel 20 und die beweglichen Teile des Spenders 11 wieder in die in Fig. 1 dargestellte Ausgangslage zurückverbracht werden.

Wird durch die Schaltnocke 27 die Rastverbindung zwischen der Trägerplatte 26 und dem Gehäuse 12 gelöst und gleichzeitig das Betätigungsmittel 20 in der zuvor erreichten Lage entweder durch den Benutzer oder aber durch Rastmittel weiter gehalten, so erfolgt nun ein Entspannen der Feder dadurch, daß die im Gehäuse 12 geführt angeordnete Trägerplatte 26 in Richtung auf die Transporttrommel 40 bewegt wird.

Während der Entspannung des Kraftspeichers 21 wird die Trägerplatte 26 mitsamt den an ihr befestigten Elementen in Richtung auf die Transporttrommel 40 bewegt. Zunächst gleitet der Schieber 44, der im wesentlichen balkenförmig ausgebildet ist und der entlang einer Kreissehne bzgl. der Transporttrommel 40 relativ beweglich ist, entlang der Stirnseite 42 der Transporttrommel 40 mit ihren Mitnehmern 43. Dabei befindet sich schon in der in den Fign. 1 und 2 dargestellten Ausgangslage des Schiebers 44 ein Mitnehmer 43 in Anlage mit der unteren Längskante 55. Dadurch, daß der Mitnehmer 43 in Anlage mit dem Schieber 44 ist, wird ein Herausziehen von Blisterstreifen auf dem Gehäuse 12 dadurch verhindert, daß ein Drehen der Transporttrommel 40 in Förderrichtung gesperrt wird.
Im weiteren Verlauf der Translationsbewegung der Trägerplatte 26 gelangt die Stirnseite 56 des Schiebers 44 in Anlage mit einem Mitnehmer 43. Ab diesem Moment ist es dem Mitnehmer 43, entlang dessen sich die untere Längskante 55 des Schiebers 44 bewegt, möglich, entweder in eine Ausnehmung des Schiebers 44 einzutauchen oder aber durch entsprechende Gestaltung der Längskante 55 unter den Schieber 44 zu gelangen. Von dem Schieber 44 angetrieben wird die Trommel über den Mitnehmer 43, der sich in Anlage mit der Stirnseite 56 des Schiebers 44 befindet, so weitergedreht, daß sich die nächste Ausnehmung 46 mit der darin gehaltenen Speicherkammer 16 des Blisterstreifens 15 in ihrer Position gegenüber dem Stoßdorn 30 befindet. Zu diesem Zeitpunkt hat sich der Mitnehmer 43 so weit weitergedreht, daß seine - in Richtung auf den Stoßdorn 30 gesehen - obere Kante unterhalb der Unterkante 55 des Schiebers 44 befindet. Dieser kann somit an dem Mitnehmer 43 vorbeigleiten, wobei ein Rückdreh-Sperreffekt dadurch entsteht, daß sich dieser Mitnehmer 43 weiterhin in Anlage mit der unteren Längskante 55 des Schiebers 44 befindet. Gleichzeitig gelangt der entgegen der Drehrichtung der Transporttrommel gesehen übernächste Mitnehmer 43 ebenfalls in Anlage an die untere Längskante 55 des Schiebers 44. Dadurch wird ein Weiterdrehen (Verdrehen) der Trommel verhindert. Nachdem sowohl ein Verdrehen in Transportrichtung der Trommel als auch in Gegenrichtung verhindert wird, ist somit eine lagesichere Ausrichtung einer Aufnahme 41 ausgebildet. Die Ausnehmung 46 und die darin gehaltene Speicherkammer 16 befindet sich in fixierter, zum Stoßdorn 30 hin ausgerichteter Lage.

Erst wenn diese Stellung der Transporttrommel 40 erreicht wird, gelangt der Schaltschieber 33 mit seiner Steuerkante 34 in Anlage mit dem Stoßdorn und den dort ausgebildeten Steuerkanten, beispielsweise dem Anlagering 35. Die Ausbildung der Steuerkante 34 des Schaltschiebers 33 ist derart, daß der Stoßdorn 30 zunächst in Richtung auf die Speicherkammer 16, die in der entsprechenden Aufnahme 41 der Transporttrommel 40 auf den Stoßdorn 30 hin ausgerichtet gehalten und entgegen der Wirkung der Blattfeder 36 bewegt wird. Danach durchstößt der Stoßdorn mit seinen hierfür ausgebildeten Kanten das die Speicherkammer 16 verschließende Material, in der Regel ein Folienmaterial, meist noch mit Metalldampf oder Ähnlichem beschichtet. Zu diesem Zeitpunkt wird auch zwischen der Pumpenkammer 22, in die der Trägerplatte 26 verbundene Pumpenkolben 57 eintaucht, und dem zweiten Strömungskanal 32 des Stoßdorns 30 eine fluidische Verbindung, beispielsweise durch Kontakt zwischen Schlauchendstücken, hergestellt. Gleichzeitig kann der Stoßdorn 30 auch als Schieber eines Schiebeventils ausgebildet sein, so daß mit Erreichen der Endlage des Stoßdorns, in der die Spitze des Stoßdorns vollständig in die Speicherkammer 16 hineinragt, der Deckel der Speicherkammer 16 durchstochen ist, jedoch noch dichtend ringförmig an dem Stoßdorn 30 anliegt, eintaucht. Das Ventil öffnet sich, wenn der Stoßdorn 30 seine Endlage erreicht hat. Dadurch kann das in der Pumpenkammer 22 komprimierte Fluid, nämlich Luft, durch den Strömungskanal 32 des Stoßdorns 30 hindurch in die Speicherkammer 16 entweichen. Dort wird das Fluid mit dem in der Speicherkammer 16 befindlichen Medium, beispielsweise einer Flüssigkeit oder einem Pulver, vermengt und tritt durch den ersten Strömungskanal, der zu der Düse 14a führt, aus.

Diese Stellung ist in der Fig. 3 dargestellt.

Nach Beendigung des Austrags wird entweder die rastende Verbindung zwischen dem Betätigungsmittel 20 und dem Gehäuse 12 gelöst oder aber der Benutzer läßt das Betätigungsmittel 20 los. Aufgrund der Wirkung der Rückstellfeder 25 und der Blattfeder 36 werden die beweglichen Teile wieder zurück in die in der Fig. 1 gezeigten Ausgangsstellung zurückgeführt. Der Spender ist dann zur nächsten Betätigung bereit.

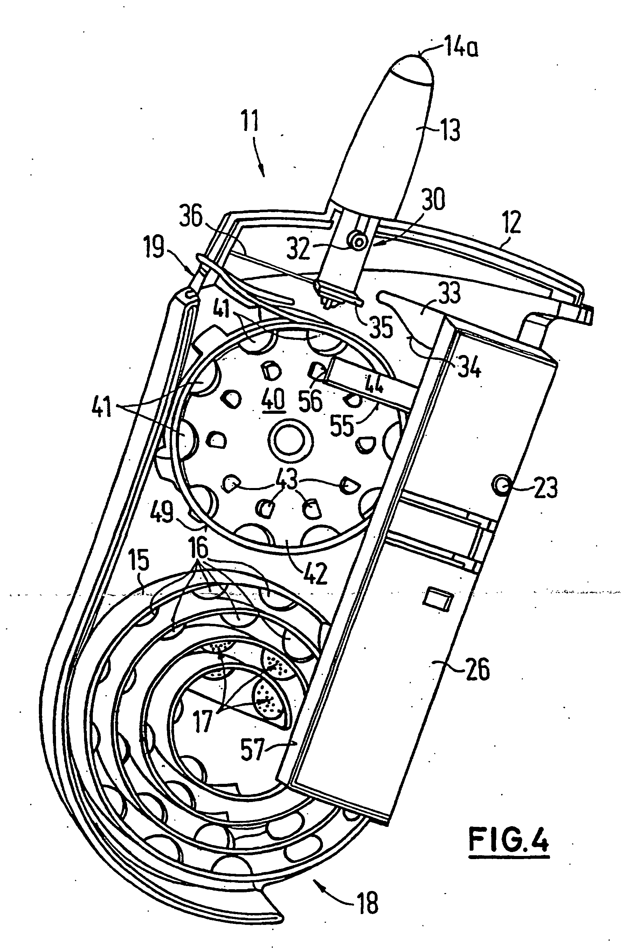

Die Fig. 4 zeigt eine alternative Ausgestaltung eines erfindungsgemäßen Spenders. Im Unterschied zu der in Fig. 1 bis 3 dargestellten Ausführungsform ist keine selbsttätige Rückführung des Betätigungsmittels 20 vorgesehen. Vielmehr muß das Betätigungsmittel 20 von dem Benutzer selbst von der der Fig. 3 entsprechenden Endlage in die der Fig. 1 entsprechenden Betätigungsausgangslage überführt werden. Ansonsten entsprechen die verschiedenen Elemente der Fig. 4 den entsprechenden Elementen in den Fign. 1 bis 3 und sind auch - soweit vorhanden - gleich nummeriert.

Der Austrag des Mediums erfolgt über die Düse 14a in den Nasenadapter 13. Der Stoßdorn 30 weist an einer Außenseite einen Anschlußstutzen für den zweiten Strömungskanal auf, der den Stoßdorn fluidisch mit der Pumpenkammer 22 verbinden kann. Der Stoßdorn 30 wird über die Blattfeder 36 in seiner Ruhelage gehalten und kann über den Schaltschieber 33, der mit der in dieser Darstellung nicht gezeigten Trägerplatte 26 verbunden ist, in seine Austragstellung gebracht werden. Hierzu gelangt im Verlauf einer Betätigung die Steuerkante 34 des Schaltschiebers 33 in Anlage mit dem Anlagering 35 am Stoßdorn.

In Form eines Trommelspeichers 18 wird ein Blisterstreifen 15 bereitgestellt. Der Blisterstreifen 15 weist eine Vielzahl von Speicherkammern 16 auf, die jeweils Medium 17 enthalten.

Der Blisterstreifen 15 ist zumindest bereichsweise über die Transporttrommel 40 geführt. Die Transporttrommel 40 weist Aufnahmen 41 für die Speicherkammern 16 des Blisterstreifens 15 auf. Dabei ist die Transporttrommel an ihrer Umfangsseite 49 mit den Aufnahmen 41 versehen. An der Stirnseite 42 weist die Transporttrommel 40 die Mitnehmer 43 auf, die als feststehend auf der Stirnseite angeordnete, von dieser in axialer Richtung abragende Erhebungen ausgebildet sind. In der dargestellten Betätigungsausgangsstellung dient der Schieber 44, der mit der Grundplatte 26 verbunden ist, sowohl als Rückdrehsperre als auch als Sperre, die ein Herausziehen des Blisterstreifens aus dem Spender verhindert, also einer Verdrehsperre. Hierzu befindet sich der Schieber 44 in der dargestellten unbetätigten Ausgangslage mit seiner Stirnseite 56 in Anlage mit einem ersten Mitnehmer 43. Zum andereren befindet sich die untere Längskante 55 in Anlage mit einem zweiten Mitnehmer 43. Die Anlage zwischen einem Mitnehmer 43 und der Stirnseite 56 verhindert ein Zurückdrehen der Transporttrommel 40. Der zweite Mitnehmer 43 wirkt aufgrund seiner Anlage mit der unteren Längskante 55 des Schiebers einem Verdrehen der Transporttrommel entgegen. Daher wird durch Schieber 44 und Trommel 40 eine komplette Verdrehsperre für die Transporttrommel 40 gebildet.

Der Funktionsablauf für einen Spender, wie er in Fig. 4 dargestellt ist, entspricht dabei vollständig dem Funktionsablauf eines Spenders in der Ausführungsform gemäß den Fign. 1 bis 3. Daher wird diesbezüglich auf die entsprechende Beschreibung zu den Fign. 1 bis 3 verwiesen.

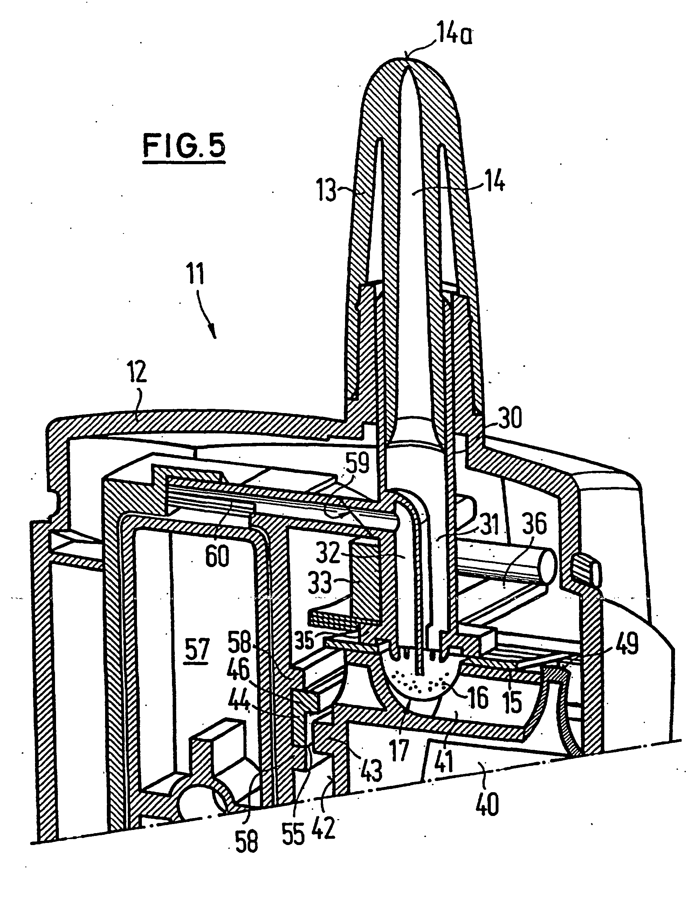

Die Fig. 5 zeigt in einer schematischen Schnittdarstellung den Stoßdorn 30 und die Strömungskanäle 31 und 32, die zum Austrag des Mediums 17 aus der Speicherkammer 16 des Blisterstreifens 15 dienen. Der Blisterstreifen 15 wird einerseits durch Führungselemente und andererseits durch die entsprechend geformten Ausnehmungen 46 an der Umfangsseite 49 der Transporttrommel 40 gehalten. An der Stirnseite 42 der Transporttrommel 40 sind die Mitnehmer 43 ausgebildet, auf die der Schieber 44 einwirken kann. Dabei weist der Schieber 44 eine Ausnehmung 46 auf, in die ein Mitnehmer 43 eintauchen kann. Dabei ist die Ausnehmung 46 so ausgebildet, daß sie sich einerseits längs der unteren Längskante 55 des Schiebers 44 erstreckt und andererseits der Stirnseite 42 der Transporttrommel 40 zugewandt ist. Damit der Schieber 44 eine lineare, einer Kreissehne der Transporttrommel 40 folgende Bewegung ausführt, sind die Führungsschienen 58 vorgesehen, in denen er geführt wird. Die Führungsschienen 58 sind im Gehäuse 12 des Spenders 11 ausgebildet.

In der dargestellten Betätigungsstellung des Spenders 11 ist der Stoßdorn 30 in die Speicherkammer 16 mit dem Medium 17 eingetaucht. Der Stoßdorn hat mit seinen Stirnkanten das Verschlußmaterial, das die Speicherkammer 16 hermetisch verschießt, durchstochen. Der zweite Strömungskanal, der zur Zufuhr von Fluid aus der Pumpenkammer 22 in die Speicherkammer 16 dient, ist über die Anschlußstelle 59 mit dem Fluidkanal 60, der von der Speicherkammer 22 zur Anschlußstelle 59 führt, verbunden worden. Nachdem das Speicherelement 21 für die Durchführung eines Hubes des Pumpenkolbens 57 erst dann sorgt, wenn diese fluidische Verbindung über die Anschlußstelle 59 hergestellt ist, erübrigt es sich, ein Schaltventil im Fluidweg zwischen Pumpenkammer 22 und zweiten Strömungskanal 32 vorzusehen. Dies wäre anders, wenn die Energie für den Austrag dadurch gespeichert würde, daß das Fluid in der Pumpenkammer komprimiert würde, bevor der Stoßdorn 30 in die Speicherkammer 16 eingedrungen ist und somit ein Austrag von Medium 17 aus der Speicherkammer 16 über den Strömungskanal 14 zur Düse 14a und damit zum Applikationsort noch nicht möglich wäre.

Das aus der Pumpenkammer 22 verdrängte Fluid gelangt über den Strömungskanal 60 und den zweiten Strömungskanal 32 des Stoßdorns 30 in die Speicherkammer 16. Dort wird es mit dem in der Speicherkammer 16 bereitgehaltenen Medium 17 vermischt. Dabei ist dies von der Form des Mediums, ob pastös, flüssig oder fest, unabhängig. Da der Stoßdorn 30 nahezu dichtend in dem Material, das die Speicherkammer verschlossen hat, abschließt, verbleibt als einziger Ausweg aus der Speicherkammer 16 für das Gemenge von Medium 17 und zugeführtem Fluid allein der erste Strömungskanal 31. Der erste Strömungskanal 31 führt zum Strömungskanal 14 in dem Nasenadapter 13 und somit zur Düse 14a. Dort erfolgt der Austrag von Fluid und Medium aus dem Spender 11.

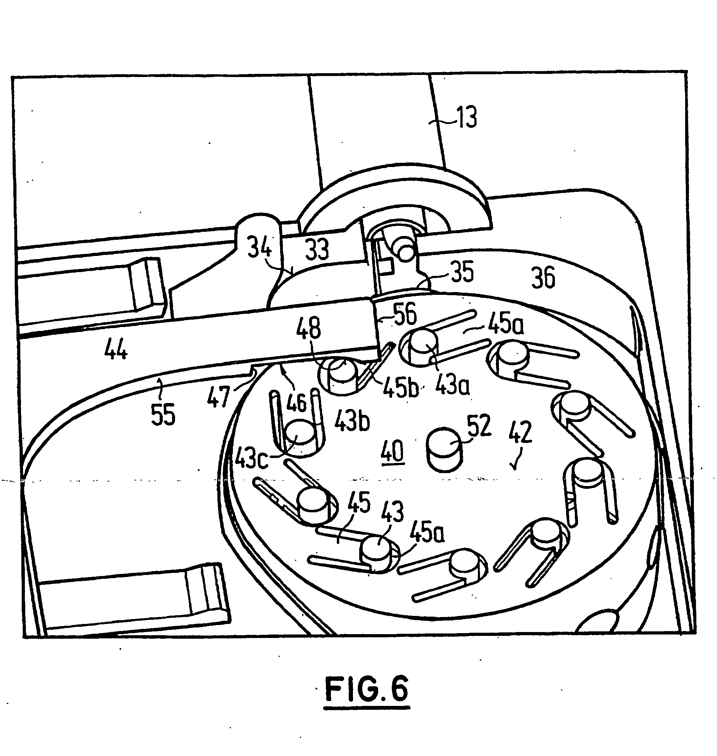

Die Fig. 6 zeigt in schematischer Darstellung, in unbetätigter Ruhestellung, eine Detailansicht von Transporttrommel 40 und Schieber 44.

Auf der Transporttrommel 40 sind Mitnehmer 43 angeordnet, die von Materialzungen 45 in axialer Richtung abragen. Dabei sind die Materialzungen 45 durch Schlitze 45a gebildet, die in die Stirnseite 42 der Transporttrommel eingebracht sind. Dabei sind die Mitnehmer 43 konzentrisch zur Drehachse 52 der Transporttrommel 40 angeordnet.

Auf der Zeichnung ebenfalls sichtbar ist der Stoßdorn 30 mit dem Anlagering 35. Dabei wird der Stoßdorn 30 einerseits in dem Nasenadapter 13 geführt und andererseits von der Blattfeder 36 in seiner unbetätigten Ruhestellung gehalten. Über den Schaltschieber 33 mit seiner Steuerkante 34 kann der Stoßdorn 30 in Richtung auf die Transporttrommel bewegt werden, wie dies vorstehend bei der Beschreibung der Betätigungsabläufe beschrieben wurde.

Bei einer Betätigung des Spenders wird der Schieber 44 in Richtung auf den obersten, ersten Mitnehmer 43a bewegt. Sobald der Schieber 44 mit seiner Stirnseite 56 in Anlage mit dem Mitnehmer 43a gelangt, führt die Transporttrommel dem weiteren Bewegungsablauf des Schiebers 44 eine Rotationsbewegung um ihre Drehachse 52 aus. Dabei gleitet der nächstfolgende, zweite Mitnehmer 43b in die Ausnehmung 46, die am Schieber 44 ausgebildet ist, sich in einem Bereich der unteren Längskante 55 des Schiebers 44 erstreckt und zur Stirnseite 42 der Transporttrommel 40 hin ausgerichtet ist. Dabei ist es möglich, die Ausnehmung 46 mit einer Anschlagfläche 47 an ihrem in Bewegungsrichtung des Schiebers 44 gesehen, hinteren Ende zu versehen. Diese Anschlagfläche 47 kann in Anlage mit dem zweiten Mitnehmer 43b gelangen und so zum einen eine Transportfunktion für die Fortsetzung der Rotationsbewegung der Transporttrommel 40 in Betätigungsrichtung und auch als Rückdrehsperre während der Betätigung des Spenders dienen. Die Anschlagfläche 47 läuft hierzu möglichst planparallel zur Stirnseite 56 des Schiebers 44, so daß der zweite Mitnehmer 43b nicht in der Lage ist, die Anschlagfläche 47 zu überfahren. Dabei wird der Bewegungsweg der Transporttrommel und des Schiebers 44 in Transportrichtung dadurch begrenzt, daß der bzgl. dem ersten Mitnehmer 43a übernächste Mitnehmer 43c in Anlage mit der unteren Längskante 55 des Schiebers 44 gelangt. Durch Anlage des Mitnehmers 43b an der Anschlagfläche 47 und der gleichzeitigen Anlage des Mitnehmers 43c an der unteren Längskante 55 wird es somit möglich, eine definierte Stellung der Transporttrommel 40 zu erzeugen, die in beide Drehrichtungen der Transporttrommel 40 verdrehgesichert ist.

Damit der Schieber 44 ohne ein Verdrehen der Transporttrommel 40 in seine Ausgangsstellung zurückbewegt werden kann, ist es vorteilhaft, eine weitere, auf die Transporttrommel 40 vorzugsweise über Mitnehmer 43 einwirkende Rückdrehsperre vorzusehen, wie sie beispielsweise in den Fign. 1 bis 3 beschrieben wurde. Darüber hinaus ist es vorteilhaft, wenn die der Anschlagfläche 47 gegenüberliegende Seite 48 der Ausnehmung 46 ebenfalls rampenförmig ausgebildet ist. Bei einer Zurückbewegung des Schiebers 44 kann die gegenüberliegende Seite 48 den Mitnehmer 43b in Richtung auf die Stirnseite 42 der Transporttrommel 40 hineindrücken. Dies geschieht entgegen der Wirkung der von der Materialzunge 45b des Mitnehmers 43b aufgrund der elastischen Verformung erzeugten Kräfte. Somit kann der Schieber 44 über den Mitnehmer 43b bei der Rückwärtsbewegung hinweg gleiten.

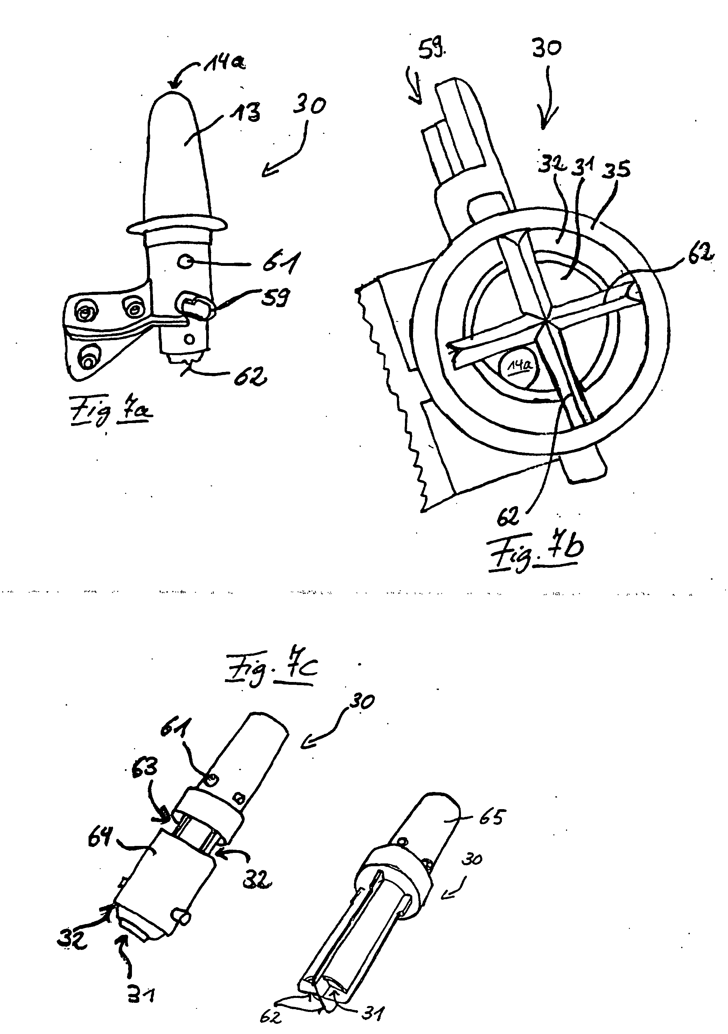

In den Figuren 7a-7c ist eine Darstellung einer alternativen Ausführungsform eines Stoßdorns 30 dargestellt, wobei in den Figuren 7a und 7b die Positionierung des Stoßdorns 30 in den Nasenadapter 13 sowie die Ausbildung der Anschlußstelle 59 des Strömungskanales 60 gezeigt ist.

Das Gehäuse des Stoßdorns umfaßt dabei einen ringförmigen Körper und eine Anschlußstelle 59, um den ringförmigen Körper mit der Pumpenkammer 22 fluidisch zu verbinden. Durch die Anschlußstelle hindurch gelangt dann ein Fluidstrom in den ringförmig ausgebildeten zweiten Strömungskanal 32 und strömt, radial gesehen an der Außenseite der Speicherkammer 16 des Blisterstreifens 15, in die Speicherkammer ein. Da der Außendurchmesser des Stoßdorns 30 an den Innendurchmesser der Speicherkammer 16 des Blisterstreifens angepasst ist, ist eine Strömung entlang der Gehäusewand der Speicherkammer 16 sichergestellt. Zum Durchstoßen der die Speicherkammer 16 abschließenden Abdeckung, weist der Stoßdorn 30 zwei rechtwinklig ausgebildete und über kreuz liegende Schneidkanten 62 auf. Die Schneidkanten können insbesondere einen im wesentlichen dreiecksförmigen Querschnitt aufweisen und sorgen dafür, daß nach dem Durchschneiden der Abdeckung ein Einführen des Stoßdorns in die Speicherkammer 16 ermöglicht wird. Dabei kann über die als Anlagering 35 ausgebildete Seitenwand des Stoßdorns 30 auch die dichtende Anlage des Stoßdorns an der Außenseite der Speicherkammer 16 sichergestellt werden.

Die kozentrisch am Rand der Speicherkammer 16 einströmende Luft entweicht über den ersten Strömungskanal 31, der kozentrisch und mittig bezüglich des zweiten Strömungskanals 32 angeordnet ist. Hierbei wird der Umlenkungseffekt ausgenutzt, der entsteht, wenn die einströmende Luft sich im tiefsten Punkt des Behälters, von allen Seiten zuströmend, trifft und dann nur in der Mitte nach oben ausweichen kann. Dadurch wird für einen guten Austrag des auszutragenden Mediums 17 aus der Speicherkammer 16 gesorgt.
In der Figur 7b ist die Ansicht des Stoßdorns 30 von unten zu sehen, bei dem der Austragkanal 31 zum Strömungskanal 14 führt, der in der Düse 14a mündet.

Darüber hinaus weist der Stoßdorn 30 an seiner Außenseite auch noch ein Überströmloch 61 auf, das mit der Atmosphäre fluidisch verbunden wird. Das Überströmloch ist mit dem zweiten Strömungskanal 32 fluidisch verbunden, wobei ein Rückschlagventil dafür sorgt, daß kein Fluid aus der Pumpenkammer 22 über das Überströmloch 61 entweicht. Das Überströmloch 61 dient dazu, daß der Benutzer den Austragvorgang dadurch unterstützen kann, daß er selber aktiv Luft ansaugt. Damit dieses aktive Ansaugen unterstützt werden kann, muß es möglich sein, daß die durch das Überstömloch 61 nachströmende Luft, die dem angesaugten Volumen des Benutzers im wesentlichen entspricht, am Austragvorgang mitwirken kann. Es kann auch vorgesehen sein, daß das Überströmloch 61 mit dem ersten Strömungskanal 31 verbunden ist und die zusätzliche Ansaugung allein durch den Venturi-Effekt des zusätzlichen Luftstromes erzielt wird. Aufgrund der hohen Strömungsgeschwindigkeit des durch das Überströmloch 61 großen Volumenstromes wird Auszutragendes Medium bzw. ein Gemisch von Fluid und Medium aus der Speicherkammer 16 herausgesaugt.

In der Figur 7c sind in perspektivischer Ansicht Teilelemente des Stoßdorns 30 gezeigt. Die über die Anschlußstelle 59 zuströmende Luft gelangt im Inneren des Gehäuses des Stoßdorns 30 an den Ringabschnitt 63, wobei zwischen der Hülse 64 und der Nadel 65, die den Stoßdorn mit den Schneidkanten 62 bildet und die ebenfalls einen hülsenförmigen Körper aufweist, die den ersten Strömungskanal 31 in ihrem Inneren aufweist durchströmt. Die zweite Teilansicht der Figur 7c zeigt die Nadel 65, die im Inneren der Hülse 64 angeordnet ist und in der der erste Strömungskanal 31 ausgebildet ist, der dem Austrag vom Medium dient. Darüber hinaus sind an der Nadel 65 die Schneidkanten 62 zum Durchtrennen der Abdeckung des Vorratsbehälters vorgesehen.

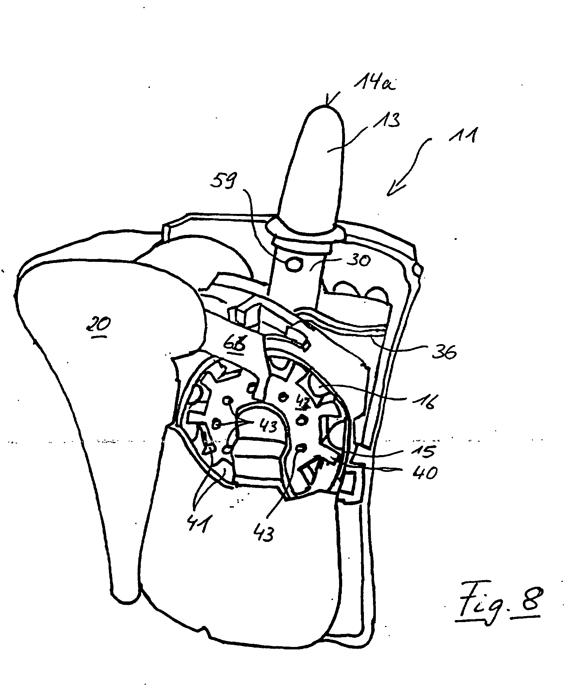

Die Figur 8 zeigt eine alternative Ausgestaltung eines Spenders.

Der Austrag des Mediums erfolgt dabei über die Düse 14a in den Nasenadapter 13. Im Inneren des Gehäuses und fluidisch verbunden mit dem Strömungskanal 14 des Nasenadapters 13 ist der Stoßdorn 30. Der Stoßdorn 30 weist dabei an seiner Außenseite eine Anschlußstelle 59 auf, über die der Stoßdorn fluidisch mit der Pumpenkammer 22 verbindbar ist. Dabei wird der Stoßdorn 30 über die Blattfeder 36 in seiner Ruhelage gehalten. Im Inneren des Gehäuses 12 des Spenders 11 sind zur Führung der Linearbewegung Führungsnocken 66 ausgebildet. Über ein nicht dargestelltes Schaltmittel, beispielsweise einen Schaltschieber, kann der Stoßdorn 30 von der Ruhelage in die dargestellte Betätigungsendlage verbracht werden.

In Form eines Trommelspeichers 18 wird der Blisterstreifen 15, der eine Vielzahl von Speicherkammern 16 aufweist, bereitgestellt. Der Blisterstreifen 15 ist dabei zumindest bereichsweise über die Transporttrommel 40 geführt, die Aufnahmen 41 für jeweils eine Speicherkammer 16 des Blisterstreifens 15 aufweist. Die Transporttrommel ist hierzu an ihrer Umfangsseite 49 mit den Aufnahmen 41 versehen. An der Stirnseite 42 weist die Transporttrommel 40 Mitnehmer 43 auf, die als in axialer Richtung abragende Erhebungen ausgebildet sind. Die Hakenkralle 66 hintergreift bei einem Herausbewegen des Betätigungsmittels 20 einen der Mitnehmer 43 und verdreht die Transporttrommel 40 bei dieser Spannbewegung um genau eine Speicherkammer 16, bzw. um genau eine Aufnahme 41. Bei der Betätigung des Betätigungsmittels 20 wird der Rückhub der Hakenkralle 66 so geführt, das die Hakenkralle 66 mit ihrem die Mitnehmer 43 hintergreifenden Element axial versetzt an den Mitnehmern 43 vorbeigeführt wird, so daß kein Zurückdrehen der Transporttrommel erfolgt. Darüber hinaus können noch entsprechende Rastmittel zur Positionshaltung der Transporttrommel 40 ausgebildet sein.

Der Funktionsablauf für die Betätigung eines Spenders gemäß Figur 8 entspricht dabei vollständig dem Funktionsablauf eines Spenders gemäß der Figuren 1 bis 3, wozu diesbezüglich auf die entsprechende Beschreibung Bezug genommen wird.

Durch erfindungsgemäße Spender können Medien vieler Ausbildungen aus einem Spender ausgetragen werden. Es ist möglich, sowohl pulverförmige als auch flüssige oder pastöse Medien auszutragen. In der Regel wird als Fluid, das aus der Pumpenkammer 22 verdrängt wird und das für den Austrag des Mediums aus der Speicherkammer sorgt, Umgebungsluft verwendet. Es können aber auch andere Fluide verwendet werden. Ferner wurde die Erfindung anhand der Anwendung bei einem Spender für den nasalen Bereich erläutert. Anstelle des Nasenadapters 13 können aber auch andere Applikatoren treten. Ein Beispiel für einen anderen Applikator wäre ein Rachenadapter. Somit kann der erfindungsgemäße Spender bei einer Vielzahl von Anwendungsfällen verwendet werden. Dies ist insbesondere bei Medien der Fall, die pharmazeutische Wirkstoffe, zum Beispiel Schmerz- oder Migränemittel oder andere, insbesondere nasal zu verabreichende Medikamente enthält. Die Verwendung des Spenders ist jedoch nicht auf den Bereich pharmazeutischer oder medizinischer Verwendung beschränkt.

## Patentansprüche

1. Spender für in Speicherkammern (16) eines Speicherkörpers portioniert verpackte, vorzugsweise wenigstens einen pharmazeutischen Wirkstoff enthaltende austragbare Medien (17), mit einem Stoßdorn (30), der in Speicherkammern (16) einführbar ist, einer Fluidpumpe mit einer Pumpenkammer (22), aus der das Fluid der Speicherkammer zum Austragen des Mediums zuführbar ist, und mit einem Betätigungsmittel (20), durch dessen Betätigung sowohl die Positionierung einer Speicherkammer (16) in Bezug auf den Stoßdorn (30) als auch das Austragen des Mediums (17) erfolgt, **gekennzeichnet, durch** ein Speicherelement (21), das während eines ersten Betätigungsabschnittes verspannbar ist und zu seiner Entspannung während eines zweiten Betätigungsabschnittes **durch** Lösen einer Verrastung (27) auslösbar ist und dabei in Folge:
- die Positionierung der Speicherkammer (16) in Bezug auf den Stoßdorn (30),
- die Öffnung der Speicherkammer (16) duch den Stoßdorn (30) mit Schaffung einer Fluidverbindung zwischen Pumpenkammer und Speicherkammer und
- die Zuführung des Fluids zur Speicherkammer (16) zum Austrag des Mediums bewirkt.

2. Spender nach Anspruch 1, **dadurch gekennzeichnet, dass** das Speicherelement (21) erst dann für einen Hub des in die Pumpenkammer (22) eingreifenden Pumpenkolbens (57) sorgt, wenn die Fluidverbindung hergestellt ist.

3. Spender nach Anspruch 1, **dadurch gekennzeichnet, daß** das Speicherelement (21) eine Pumpenkammer ist, wobei während des ersten Betätigungsabschnittes in der Pumpenkammer ein Druck aufgebaut wird, wobei die Pumpenkammer durch ein schaltbares Ventil verschlossen und beim Übergang vom ersten zu dem zweiten Betätigungsabschnitt das Ventil abhängig vom Betätigungsweg des Betätigungsmittels (20) im Öffnungssinne betätigt wird.

4. Spender nach einem der vorhergehenden Ansprüche, **dadurch gekennzeichnet, daß** das austragende Medium durch Ausblasen des in der Pumpenkammer (22) enthaltenen Fluids erfolgt.

5. Spender nach einem der vorhergehenden Ansprüche, **dadurch gekennzeichnet, daß** am Stoßdorn (30) wenigstens ein erster Strömungskanal (31) zum Austrag des Mediums (17) aus dem Speicherkörper (16) und ein zweiter Strömungskanal (32) zur Zufuhr des Fluids aus der Pumpenkammer (22) in die Speicherkammer (16) ausgebildet ist.

6. Spender nach Anspruch 5, **dadurch gekennzeichnet, daß** der Stoßdorn (30) als Schaltelement des die Pumpenkammer (22) abschließende Ventils ausgebildet ist.

7. Spender nach Anspruch 6, **dadurch gekennzeichnet**
**daß** der zweite Strömungskanal (32) ringförmig ausgebildet ist und den ersten Strömungskanal (31) umgibt.

8. Spender nach einem der vorhergehenden Ansprüche, **dadurch gekennzeichnet, daß** die Speicherkammer (16) durch ein Folienmaterial verschlossen ist und der Stoßdorn (30) derart ausgebildet ist, daß der Rand des Stoßdorns (30) dichtend an dem von ihm durchstoßenen Folienmaterial anliegt.

9. Spender nach einem der vorhergehenden Ansprüche, **dadurch gekennzeichnet, daß** der Spender (11) eine Transporttrommel (40) zum Transportieren und Ausrichten der Speicherkammern (16) aufweist, wobei die Transporttrommel (40) an ihrer Umfangsfläche (49) Aufnahmen (41) für jeweils eine Speicherkammer (16) aufweist.

10. Spender nach Anspruch 9, **dadurch gekennzeichnet, daß** eine auf die Transporttrommel (40) einwirkende Verdrehsperre vorgesehen ist, wobei durch die Wirkung der Verdrehsperre eine Aufnahme (41) für eine Speicherkammer (16) in ihrer bzgl. dem Stoßdorn (30) ausgerichteten Lage gehalten ist.

11. Spender nach Anspruch 9 oder 10, **dadurch gekennzeichnet, daß** eine auf die Transporttrommel (40) einwirkende Rückdrehsperre ausgebildet ist, die eine Drehbewegung der Transporttrommel (40) in einer Transportrichtung erlaubt und eine Drehbewegung entgegen der Transportrichtung sperrt, wobei die Rückdrehsperre vorzugsweise in eine leere Aufnahme (41) eingreift.

12. Spender nach einem der Ansprüche 9 bis 11, **dadurch gekennzeichnet, daß** die Transporttrommel(40) an einer Stirnseite (42) Mitnehmer (43, 43a, 43b, 43c) aufweist, wobei vorzugsweise jeder Aufnahme (41) der Umfangsfläche (49) stirnseitig ein Mitnehmer (43) zugeordnet ist.

13. Spender nach Anspruch 12, **dadurch gekennzeichnet, daß** das Betätigungsmittel (20) wenigstens einen auf Mitnehmer (43) einwirkenden Schieber (44) aufweist, wobei der Schieber (44) zugleich zumindest entweder als Verdrehsperre oder als Rückdrehsperre ausgebildet ist.

14. Spender nach Anspruch 13, **dadurch gekennzeichnet, daß** der Schieber (44) mittels dem Betätigungsmittel (20) verschiebbar und so geführt angeordnet ist, daß er auf einer Kreissehne entlang der Stirnseite (42) an der Transporttrommel 40) vorbeibewegt wird.

15. Spender nach einem der Ansprüche 12 bis 14, **dadurch gekennzeichnet, daß** die Mitnehmer (43) als in axialer Richtung von der Stirnseite (42) abragende, vorzugsweise zylindrisch ausgebildete, Erhebungen ausgebildet sind, die konzentrisch zur Drehachse (52) der Transporttrommel angeordnet sind

16. Spender nach Anspruch 15, **dadurch gekennzeichnet, daß** die Mitnehmer (43) auf in der Stirnseite (42) ausgebildeten Materialzungen (45) angeordnet sind, wobei die Materialzungen (45) vorzugsweise U-förmig ausgebildet sind, in Drehrichtung der Transporttrommel der Stirnseite (42) der Transporttrommel über einen Materialsteg verbunden sind und derart ausgebildet sind, daß sie um die Höhe der jeweiligen Erhebung entgegen der dabei entstehenden Biegekräfte in Richtung auf das Innere der Transporttrommel (40) verbringbar sind.

17. Spender nach einem der Ansprüche 12, 15 oder 16, **dadurch gekennzeichnet, daß** das Betätigungsmittel (20) eine Hakenkralle (66) aufweist, wobei die Hakenkralle (66) zum Transportieren der Transporttrommel (40) mit wenigstens einem Mitnehmer (43) in Eingriff gelangt und bei der Rückbewegung axial versetzt an wenigstens einem Mitnehmer (43) vorbei zurückgeführt wird.

18. Spender nach einem der Ansprüche 14 bis 16, **dadurch gekennzeichnet, daß** der Schieber (44) im wesentlichen balkenförmig ausgebildet ist, wobei sich seine Längskante (55) in Richtung der Kreissehne entlang derer der Schieber bzgl. der Transporttrommel (40) beweglich ist, erstreckt, wobei seine Stirnseite (56) auf einen Mitnehmer (43, 43a) hin ausgerichtet ist, wobei der Schieber (44) während eines Betätigungsvorganges mit seiner Stirnseite (56) in Anlage mit einem Mitnehmer (43, 43a) gelangt; daß der Schieber (44) eine Ausnehmung (46) ausweist, die auf seiner der Stirnseite der Transport-trommel (40) zugewandten Seite ausgebildet ist und die sich auch in den Bereich der den Mitnehmern (43) zuge-wandten Längskante (55) erstreckt, wobei die Ausnehmung (46) der Aufnahme von dem Mitnehmer (43, 43b) dient, der auf den Mitnehmer (43, 43a) folgt, welcher bei einer Betätigung mit der Stirnseite (56) des Schiebers (44) in Anlage gelangt; daß die in Bewegungsrichtung hintere, parallel zur Stirnseite (56) des Schiebers (44) ausgerichtete, die Ausnehmung (46) begrenzende Anschlagfläche (47) in ihrer Lage bzgl. der Stirnseite (56) und den Mitnehmern (43) derart ausgerichtet ist, daß eine in Transportrichtung der Transporttrommel (40) gerichtete kraftschlüssige Verbindung zwischen einem in der Aufnahme befindlichen Mitnehmer (43, 43b) und Anschlagfläche (47) herstellbar ist und daß die Ausnehmung (46) an der in Bewegungsrichtung vorderen, der Anschlagfläche (47) gegenüberliegenden Seite (48), die die Ausnehmung begrenzt, rampenförmig ausgebildet ist.

19. Spender nach einem der Ansprüche 9 bis 18, **dadurch gekennzeichnet, daß** eine auf die Transporttrommel (40) einwirkende, vorzugsweise dem Schieber (44) diametral gegenüberliegende Rückdrehsperre vorgesehen ist, deren Sperrwirkung durch Kraftschluß zwischen einem transporttrommelseitigen Mitnehmer (43) und einer gehäusefesten Anlagefläche (51) erreicht wird, wobei die Anlagefläche (51) in Transportrichtung der Transporttrommel (40) überfahrbar ist.

20. Spender nach einem der vorhergehenden Ansprüche, **dadurch gekennzeichnet, daß** der Speicher (15) ein Blisterstreifen ist, der eine Mehrzahl von Speicherkammern (16) mit austragbarem Medium (17) aufweist, wobei der Blisterstreifen in einem Trommelspeicher (18) bereitgestellt ist.

## Claims

1. Dispenser for dischargeable media (17) containing preferably at least one pharmaceutical substance and packed in portioned manner in storage chambers (16) of a storage body, having a punch (30) introduceable into the storage chamber (16), a fluid pump with a pump chamber (22) from which the fluid can be supplied to the storage chamber for the discharge of the medium, and with an actuating means (20) through the actuation of which both the positioning of a storage chamber (16) relative to the punch (30) and the discharge of the medium (17) takes place, **characterized by** a storage element (21), which is tensionable during a first actuating portion and for its relief during a second actuating portion can be released by releasing a locking means (27) with the following sequence:
- the positioning of the storage chamber (16) relative to the punch (30),
- the opening of the storage chamber (16) by punch (30) creating a fluid connection between the pump chamber and the storage chamber and
- the supply of fluid to the storage chamber (16) brings about the discharge of the medium.

2. Dispenser according to claim 1, **characterized in that** the storage element (21) only ensures a stroke of the pump piston (57) engaging in the pump chamber (22) when the fluid connection has been made.

3. Dispenser according to claim 1, **characterized in that** the storage element (21) is a pump chamber and a pressure is built up in the pump chamber during the first actuating phase, in which the pump chamber is closed by an operable valve and during the transition from the first to the second actuating phase the valve is actuated in the opening sense dependent on the actuating path of the actuating means (20).

4. Dispenser according to one of the preceding claims, **characterized in that** the medium is discharged by blowing out the fluid contained in the pump chamber (22).

5. Dispenser according to one of the preceding claims, **characterized in that** on the punch (30) is formed at least one first flow channel (31) for discharging the medium (17) from the storage means (16) and a second flow channel (32) for the supply of the fluid from the pump chamber (22) into the storage chamber (16).

6. Dispenser according to claim 5, **characterized in that** the punch (30) is constructed as an operating element for the valve sealing the pump chamber (22).

7. Dispenser according to claim 6, **characterized in that** the second flow channel (32) is constructed in annular manner and surrounds the first flow channel (31).

8. Dispenser according to one of the preceding claims, **characterized in that** the storage chamber (16) is closed by a foil material and the punch (30) is constructed in such a way that the edge of said punch (30) engages in sealing manner on the foil material to be perforated by it.

9. Dispenser according to one of the preceding claims, **characterized in that** the dispenser (11) has a conveying drum (40) for conveying and orienting the storage means (16), in which the circumferential surface (49) of the conveying drum (40) carries receptacles (41) for in each case one storage chamber (16).

10. Dispenser according to claim 9, **characterized in that** a twist preventer acting on the conveying drum (40) is provided and as a result of the action of the twist preventer a receptacle (41) for a storage chamber (16) is kept in its position oriented with respect to the punch (30).

11. Dispenser according to claim 9 or 10, **characterized in that** a reverse preventer acting on the conveying drum (40) is provided, which allows a rotary movement of the conveying drum (40) in a conveying direction and prevents a rotary movement counter to the conveying direction, the reverse preventer preferably engaging in an empty receptacle (41).

12. Dispenser according to one of the claims 9 to 11, **characterized in that** on its end face (42) the conveying drum has dogs (43, 43a, 43b, 43c) and preferably a dog (43) is endwise associated with each receptacle (41) of the circumferential surface (49).

13. Dispenser according to claim 12, **characterized in that** the actuating means (20) has at least one slide (44) acting on the dogs (43), the slide (44) being simultaneously constructed as a twist preventer or as a reverse preventer.

14. Dispenser according to claim 13, **characterized in that** the slide (44), by means of the actuating means (20), can be slid and guided in such a way that along a circular chord along the end face (42) it is moved past the conveying drum (40).

15. Dispenser according to one of the claims 12 to 14, **characterized in that** the dogs (43) are constructed as preferably cylindrical protuberances projecting axially from the end face (42) and which are positioned concentrically to the rotation axis (52) of the conveying drum.

16. Dispenser according to claim 15, **characterized in that** the dogs (43) are located on material tongues (45) formed in the end face (42), the material tongues (45) preferably being U-shaped, are connected by means of a material web in the rotation direction of the conveying drum and are constructed in such a way that they can be brought by the height of the particular protuberance and counter to the resulting bending forces in the direction of the interior of the conveying drum (40).

17. Dispenser according to one of the claims 12, 15 or 16, **characterized in that** the actuating means (20) have a hook claw (66), which for the conveying of the conveying drum (40) engages with at least one dog (43) and during the return movement is moved past at least one dog (43) in axially displaced manner.

18. Dispenser according to one of the claims 14 to 16, **characterized in that** the slide (44) has a substantially beam-like construction and its longitudinal edge (55) extends in the direction of the circular chord along which the slide can be moved with respect to the conveying drum (40), its end face (56) being oriented towards a dog (43, 43a) and during an actuating process the end face (56) of the slide (44) engages with a dog (43, 43a), that the slide (44) has a recess (46) constructed on its side facing the end face of the conveying drum (40) and which extends in the area of the longitudinal edge (55) facing the dogs (43), the recess (46) serving to receive the dog (43, 43b) following the dog (43, 43a) which engages with the end face (56) of the slide (44) during an actuation, that the rear stop face (47) in the movement direction, which is oriented parallel to the end face (56) of the slide (44) and which bounds the recess (46) is so oriented in position with respect to the end face (56) and dogs (43) that a non-positive connection directed towards the conveying direction of the conveying drum (40) can be produced between a dog (43, 43b) located in the receptacle and the stop face (47) and that on the front side (48), which bounds the recess and faces the stop face (47), the recess (46) has a ramp-like construction.

19. Dispenser according to one of the claims 9 to 18, **characterized in that** a reverse preventer acting on the conveying drum (40) and preferably diametrically facing the slide (44) is provided and its blocking action is achieved by frictional connection between a conveying drum-side dog (43) and a casing-fixed contact face (51), over which it is possible to pass in the conveying direction of the conveying drum (40).

20. Dispenser according to one of the preceding claims, **characterized in that** the store (15) is a blister strip having a plurality of storage chambers (16) with dischargeable medium (17), the blister strip being made available in a drum store (18).

## Revendications

1. Distributeur pour des substances (17) à décharger, contenant de préférence au moins un produit pharmaceutique, emballées de manière préportionnée dans des chambres à réservoir (16) d'un corps de réservoir, avec un poinçon (30), qui peut être introduit dans les chambres à réservoir (16), une pompe à fluides, présentant une chambre de pompe (22), à partir de laquelle le fluide peut être amené dans la chambre à réservoir pour décharger la substance, et avec un moyen d'actionnement (20) par l'actionnement duquel a lieu non seulement le positionnement d'une chambre à réservoir (16) par rapport au poinçon (30), mais aussi la décharge de la substance (17), **caractérisé par** un élément de réservoir (21), qui peut être tendu pendant une première phase d'actionnement et qui peut être déclenché pendant une deuxième phase d'actionnement en relâchant un moyen d'encliquetage (27) en produisant consécutivement :
- le positionnement de la chambre à réservoir (16) par rapport au poinçon (30),
- l'ouverture de la chambre à réservoir (16) par le poinçon (30) en créant un raccord fluidique entre la chambre de pompe et la chambre à réservoir et
- l'amenée du fluide vers la chambre à réservoir (16) pour la décharge de la substance.

2. Distributeur d'après la revendication 1, **caractérisé en ce que** l'élément de réservoir (21) pourvoit à ce que une course du piston de pompe (57) s'engageant dans la chambre de pompe (22) puisse être produite uniquement quand le raccord fluidique a été créé.

3. Distributeur d'après la revendication 1, **caractérisé en ce que** l'élément de réservoir (21) est une chambre de pompe, une pression étant établie dans la chambre de pompe pendant la première phase d'actionnement, la chambre de pompe étant fermée par une soupape à commande, la soupape pouvant être actionnée dans le sens d'une ouverture, au passage de la première à la deuxième phase d'actionnement en fonction de la course d'actionnement du moyen d'actionnement (20).

4. Distributeur d'après une des revendications précédentes, **caractérisé en ce que** la décharge de la substance est effectuée en évacuant le fluide contenu dans la chambre de pompe (22) en soufflant.

5. Distributeur d'après une des revendications précédentes, **caractérisé en ce qu'**on réalise au moins un premier conduit d'écoulement (31) pour la décharge de la substance (17) hors du corps de réservoir (16) et un deuxième conduit d'écoulement (32) pour l'amenée du fluide de la chambre de pompe (22) dans la chambre à réservoir (16).

6. Distributeur d'après la revendication 5, **caractérisé en ce que** le poinçon (30) est réalisé en tant qu'élément de commande de la soupape qui ferme la chambre de pompe (22).

7. Distributeur d'après la revendication 6, **caractérisé en ce que** le deuxième conduit d'écoulement (32) est réalisé de manière annulaire et qu'il entoure le premier conduit d'écoulement (31).

8. Distributeur d'après une des revendications précédentes, **caractérisé en ce que** la chambre à réservoir (16) est fermée par un matériau sous forme de pellicule et que le poinçon (30) est réalisé de telle manière que le bord du poinçon (30) est adjacent de façon étanchante au matériau sous forme de pellicule qu'il a percé.

9. Distributeur d'après une des revendications précédentes, **caractérisé en ce que** le distributeur (11) présente un tambour de transport (40) pour le transport et l'alignement des chambres à réservoir (16), le tambour de transport (40) présentant à sa surface de circonférence (49) des logements (41) pour une chambre à réservoir (16) respectivement.

10. Distributeur d'après la revendication 9, **caractérisé en ce qu'**on prévoit un arrêt de mouvement angulaire agissant sur le tambour de transport (40), un logement (41) pour une chambre à réservoir (16) étant maintenu dans sa position par rapport au poinçon (30) par l'effet de l'arrêt de mouvement angulaire.

11. Distributeur d'après la revendication 9 ou 10, **caractérisé en ce qu'**on réalise un dispositif antiretour agissant sur le tambour de transport (40), qui permet un mouvement de rotation du tambour de transport (40) dans une direction de transport et qui empêche un mouvement de rotation dans le sens opposé à la direction de transport, le dispositif antiretour s'engageant de préférence dans un logement (41) vide.

12. Distributeur d'après une des revendications de 9 à 11, **caractérisé en ce que** le tambour de transport (40) présente à une face frontale (42) des tocs d'entraînement (43, 43a, 43b, 43c), un toc d'entraînement (43) étant associé de préférence à chaque logement (41) de la surface de circonférence (49) du côté frontal.

13. Distributeur d'après la revendication 12, **caractérisé en ce que** le moyen d'actionnement (20) présente au moins une barre (44) agissant sur des entraîneurs (43), la barre (44) étant réalisée de façon équivalente au moins en tant que arrêt de mouvement angulaire ou en tant que dispositif antiretour.

14. Distributeur d'après la revendication 13, **caractérisé en ce que** la barre (44) est disposée de manière déplaçable par le dispositif d'actionnement (20) et qu'elle est guidée de manière à passer sur une corde de cercle le long de la face frontale (42) à côté du tambour de transport (40).

15. Distributeur d'après une des revendications de 12 à 14, **caractérisé en ce que** les tocs d'entraînement (43) sont réalisés en tant que saillies de préférence cylindriques, qui se dressent en direction axiale de la face frontale (42) et qui sont disposées de manière concentrique par rapport à l'axe de rotation (52) du tambour de transport.

16. Distributeur d'après la revendication 15, **caractérisé en ce que** les tocs d'entraînement (43) sont disposés sur des languettes (45) réalisées dans la face frontale (42), les languettes (45) étant réalisées de préférence en forme de U, raccordées par une nervure en direction de rotation du tambour de transport de la face frontale (42) du tambour de transport et réalisées de telle manière qu'elles peuvent être déplacées en direction de l'intérieur du tambour de transport (40), contre la force de flexion qui en résulte, d'une hauteur correspondant à la respective saillie.

17. Distributeur d'après une des revendications 12, 15 ou 16, **caractérisé en ce que** le dispositif d'actionnement (20) présente une griffe à crochet (66), la griffe à crochet (66) se mettant en prise avec au moins un toc d'entraînement (43) pour le transport du tambour de transport (40) et étant ramenée en position axialement décalée pendant le mouvement de rappel, en passant à côté au moins d'un toc d'entraînement (43).

18. Distributeur d'après une des revendications de 14 à 16, **caractérisé en ce que** la barre (44) est réalisée essentiellement à la manière d'un bâton, son arête longitudinale (55) s'étendant en direction de la corde de cercle, le long de laquelle la barre est mobile par rapport au tambour de transport, sa face frontale (56) étant orientée en direction d'un toc d'entraînement (43, 43a), la barre (44) se mettant en contact par sa face frontale (56) avec un toc d'entraînement (43, 43a) pendant la mise en action ; **en ce que** la barre (44) présente un creux (46) qui est réalisé à sa face orientée vers la face frontale du tambour de transport (40) et qui s'étend aussi dans le domaine de l'arête longitudinale (55) orientée vers les tocs d'entraînement (43), le creux (46) servant au logement du toc d'entraînement (43, 43b), qui fait suite au toc d'entraînement (43, 43a), qui se met en contact avec la face frontale (56) de la barre (44) pendant un actionnement ; **en ce que** la surface d'arrêt (47), postérieure en direction de mouvement, qui est orientée parallèlement à la face frontale (56) de la barre (44) et qui délimite le creux (46), est ajustée dans sa position par rapport à la face frontale (56) et aux tocs d'entraînement (43) de telle manière qu'on peut produire un raccord à engagement positif orienté en direction de transport du tambour de transport (40) entre un toc d'entraînement (43, 43b) situé dans le logement et la surface d'arrêt (47) et **en ce que** le creux (46) à la face (48) délimitant le creux, antérieure en direction de mouvement et opposée à la surface d'arrêt (47) est réalisé de manière à présenter une déclivité linéaire.

19. Distributeur d'après une des revendications de 9 à 18, **caractérisé en ce qu'**on prévoit un dispositif antiretour agissant sur le tambour de transport (40), situé de préférence de façon diamétralement opposée à la barre (44), dont l'effet de blocage est obtenu par l'adhérence entre un toc d'entraînement (43) du côté du tambour de transport et une surface d'arrêt (51) fixe avec le boîtier, la surface d'arrêt (51) pouvant être outrepassée en direction de transport du tambour de transport (40).

20. Distributeur d'après une des revendications précédentes, **caractérisé en ce que** le réservoir (15) est un ruban d'emballage sous blister, qui présente un certain nombre de chambres à réservoir (16) avec une substance (17) à décharger, le ruban d'emballage sous blister étant mis à disponibilité dans un réservoir à tambour (18).
